# EUROPEAN PATENT SPECIFICATION

(11) **EP 2 729 089 B1**
(45) Date of publication and mention of the grant of the patent: **02.03.2016**
(21) Application number: 12733484.5
(22) Date of filing: 06.07.2012
(51) Int. Cl.: A61F 2/28, A61C 8/00

(54) **BONE AUGMENTATION IN DENTAL IMPLANTOLOGY**
KNOCHENAUFBAU IN DER ZAHNIMPLANTOLOGIE
AUGMENTATION OSSEUSE EN IMPLANTOLOGIE DENTAIRE

(30) Priority: 08.07.2011 DE 102011051713
(43) Date of publication of application: 14.05.2014
(73) Proprietor: Giesenhagen, Bernd, 34212 Melsungen (DE); Yüksel, Orcan, 60320 Frankfurt am Main (DE)
(72) Inventor: GIESENHAGEN, Bernd, 34212 Melsungen (DE)
(74) Representative: Dennemeyer & Associates S.A.
(86) International application number: PCT/EP2012/063311
(87) International publication number: WO 2013/007657

(56) References cited:
- EP-A2- 1 508 311
- WO-A1-00/35510
- WO-A1-2011/075800
- DE-A1- 19 705 571
- DE-A1-102009 028 824
- DE-A1-102011 016 279
- US-A1- 2009 220 915
- US-B1- 6 309 220

## Description

### Field of the invention

The invention relates to a bone transplant.

The invention is used in the area of implantology. Implantology is a branch of odontology concerned with the insertion of implants or dental implants. An implant is understood as meaning a foreign body inserted into the jawbone. By being usable as a carrier of a tooth replacement, dental implants assume the function of artificial tooth roots. This generally involves them being screwed or inserted into the jawbone. Within 3 to 6 months, they bond with the surrounding bone to form a solid carrier unit capable of withstanding loads.

A precondition for introducing an implant is, however, that the jawbone is intact, or at least sufficiently present. After serious accidents, this may not be so. In such cases, before an implant is introduced, the jawbone must be re-formed (this is referred to as augmentation). For this purpose, bone material is transplanted. A bone transplant restores the jawbone locally, in order to be able to subsequently fix an implant in the bone transplant in the jawbone.

Therefore, in recent years, the procedure followed for tooth replacement treatment in the case of a badly damaged jawbone has been as follows:
A bone transplant in the form of a bone ring (to be more precise a hollow cylinder of bone) is prepared for receiving the implant. For receiving the bone ring in the jawbone, the latter is drilled out cylindrically to a suitable size. The bone ring is inserted into the drilled-out location.

Multi-part dental implants have become most widely used. Multi-part implants consist of the body, which is anchored in the bone, the neck part lying in the region of the mucous membrane of the mouth, and the head part, which receives the superstructure (e.g. a crown as a tooth replacement). The three parts are generally screwed to one another.

The body of the implant together with the neck part are subsequently screwed into the bone ring. In order that the bone ring does not turn together with the body of the implant when the latter is screwed in, the bone ring is held for a short time by a holding instrument.

The drilled-out location in the bone ring has a slightly smaller diameter than the body of the implant to be screwed in. This produces a high primary stability of the body of the implant in the bone ring.

After that, the gum is sewn over the wound.

After ten days, the healing of the wound on the gum is usually complete. Depending on the bone quality, this is followed by a phase of incorporation without loading. This phase takes, on average, 2-3 months in the lower jaw and 5-6 months in the upper jaw, since the bone density of the lower jaw and the upper jaw differs. For this transitional time, a temporary tooth replacement is used.

In the case of a multi-part implant, after the phase of incorporation, the thread of the implant body is exposed again, the implant neck and head parts are screwed in and the superstructure is produced and fitted.

A distinction is made between transplants of the following types:
- autologous or autogenous transplantation: the donor and the recipient are the same person, in which case the transplant is known as autoplasty,
- syngeneic or isogeneic transplantation: the donor is an identical twin, i.e. the donor and recipient are genetically identical,
- allogeneic transplantation: the donor is a different individual, but of the same species; the transplant is known as an allograft,
- xenogeneic transplantation: the donor is of a different species (e.g. bovine bone); the transplant is known as a xenograft.

### Prior art

It has been customary until now to source the bone ring for the augmentation of the jawbone from the patient itself, for instance from a different location in the jawbone. Such procedures are e.g. described in Khoury (Int J Oral Maxillofac Implants. 1999 Jul-Aug; 14(4):557-64). This causes a further wound for the patient.

In order to avoid this, in some cases foreign bones are also used for the augmentation. Suitable as a basis for obtaining the foreign bone are e.g. bony structures that are extracted during the implantation of a joint prosthesis (allograft). The use of such allografts is described e.g. by Novell et al. (Implant Dent. 2012 Apr; 21(2):129-35). However, animal bones also come into consideration as a starting material (xenograft), as reported e.g. by Aldredge & Nejat (Compend Contin Educ Dent. 2011 May; 32 (4) :66-71) .

In order to avoid a rejection reaction from the recipient during the implantation of the bone transplant, the proteins contained in the bony structures are extracted to the greatest extent possible. Only some collagen is left in the bone, so that it retains a desired elasticity. What remains is an otherwise mineral matrix; see e.g. Gapski et al. (Int J Periodontics Restorative Dent. 2006 Feb; 26(1):59-69).

The prior art closest to claim 1 is disclosed in DE 10 2009 028 824.

### Problem

The problem addressed by the invention is that of improving the possibilities of jawbone formation in implantology.

### Solution

This problem is solved by the inventions with the features of the independent claim. Advantageous developments of the inventions are characterized in the dependent claims. The wording of all the claims is hereby incorporated into this description by reference.

To solve the problem, an allogeneic or xenogeneic bone transplant with a clearance, into which an implant can be introduced, is proposed. The bone transplant is formed such that it can be inserted into a jawbone for augmentation. In this case, the bone transplant is designed such that it can be expanded by introducing the implant into the clearance.

The bone transplant may be formed, for example, such that it is hollow-cylindrical, frustoconical or rotationally symmetrical with respect to the axis of the clearance, which is the axis of the introduction of the implant. The clearance may pass completely through the bone transplant, but does not have to.

Such a bone transplant can be commercially prefabricated and offered. This avoids the disadvantage of the previous bone augmentation, in which the doctor carrying out the treatment had to obtain the bone ring from bone of the patient or the allograft or xenograft, or had to shape it, during the operation. In such a case, the doctor was often under great time pressure. Furthermore, under these conditions it was only possible with difficulty to realize more complex geometries, such as those proposed here. This is where the invention comes in.

A quicker and better connection between the bone transplant and the cut-out jawbone is achieved if the bone transplant has at least one expansion joint. In this case, the expansion joint is arranged such that the bone transplant can be made to expand by introducing the implant into the clearance.

The expansion joint must be formed such that the bone transplant can expand as a result of the formation of the joint. The expansion joint will therefore extend transversely in relation to the direction in which the implant is introduced from the outside up to the clearance and be open towards at least one end of the bone transplant. The expansion joint will typically extend along at least part of the clearance, generally parallel to the direction in which the implant is introduced (longitudinal axis).

The expansion joint may at one point separate the bone transplant along its longitudinal axis completely or only partially.

In the first-mentioned case, the screwing in of an implant of which the outside diameter is slightly greater than the inside diameter of the clearance has the effect that the bone transplant is made to expand. It therefore bonds better to the surrounding jawbone.

The expansion joint is in this case only formed at one point, in order that the bone transplant remains in one piece.

In a further development, four expansion joints may be formed, which do not extend along the entire length of the bone transplant, the expansion joints beginning from the end opposite to the opening for introducing the implant.

A further improvement of the bonding between the bone transplant and the jawbone is achieved if the clearance has a region tapering in the direction in which the implant is introduced. If the clearance is formed cylindrically with a circular cross section, which is usually likely to be the case, in this development the cross-sectional area tapers in the direction in which the implant is introduced. The insertion of a substantially cylindrical implant then makes the bone transplant expand and presses it against the wall of the milled-out region in the jawbone.

In this development, at least one expansion joint may be formed, from the end opposite to the opening for introducing the implant, so as to begin at least partially in the tapering region. This leads to the bone transplant acting in the manner of a straddling dowel, since it is effectively pressed apart by introducing a substantially cylindrical implant.

The described bone transplant is used in the augmentation of a jawbone.

The problem is also solved by a set with a plurality of allogeneic or xenogeneic bone transplants. The bone transplants each have a clearance into which an implant can be introduced. They are formed in such a way that they can be inserted into a jawbone for augmentation. The geometrical form of at least a first of the bone transplants differs from the geometrical form of at least a second of the bone transplants.

If the bone transplants have the form of hollow cylinders, a set may include various hollow cylinders, in which case the three typical parameters for describing a hollow cylinder are varied. These parameters are the length of the hollow cylinder, the inside diameter of the clearance and the outside diameter of the hollow cylinder. Hollow cylinders of different lengths or hollow cylinders with different outside diameters may be provided, for example.

In this way, the doctor carrying out the treatment can be provided with the typically required sizes of bone transplants, in particular bone rings, optionally also with more complex geometries, ready-prepared for the operation. For the doctor carrying out the treatment, the workload is reduced and the operating procedure is accelerated.

With such a prefabricated set of bone transplants, the doctor carrying out the treatment can also be provided in particular with the special configurations of the bone transplants with their improved bonding in the jawbone that are described above. He then finds not only bone transplants of the required size but also with the particularly advantageous geometries that he would not be able to produce himself during the operation.

It is generally of interest if the geometrical form of at least a first of the bone transplants represents a scaling of the geometrical form of at least a second of the bone transplants, that is to say if the set comprises bone transplants of different sizes. Scaling in this case refers to a proportional change in size.

The sets of the various bone transplants are used in the augmentation of jawbones.

Also disclosed is a method for augmenting a jawbone. In what follows, individual steps of this method will be described in more detail. The steps do not necessarily have to be performed in the order given in the text. Also, further steps not explicitly stated may be part of the method. The method for augmenting a jawbone comprises the following steps:
An allogeneic or xenogeneic bone transplant as described above is provided. A cylindrical recess of a size suitable for inserting the bone transplant into the jawbone is drilled out in said jawbone. The bone transplant is subsequently inserted into the drilled out recess in the jawbone. A dental implant is introduced into the clearance of the bone transplant, thus expanding the bone transplant. It therefore bonds better to the surrounding jawbone.

Further details and features emerge from the following description of preferred exemplary embodiments in conjunction with the dependent claims. The respective features may be realized here on their own or together in combination. The possibilities of solving the problem are not restricted to the exemplary embodiments.

The exemplary embodiments are schematically represented in the figures. The same reference numerals in the individual figures thereby designate elements that are the same or functionally the same or correspond to one another with regard to their functions. Specifically:
- Fig. 1: shows a longitudinal section of a bone transplant;
- Fig. 2: shows a first bone transplant in a perspective representation;
- Fig. 3: shows the cross sections A-A and B-B for the first bone transplant;
- Fig. 4: shows the cross sections A-A and B-B for a second bone transplant;
- Fig. 5: shows a third bone transplant;
- Fig. 6: shows the cross section A-A of the third bone transplant;
- Fig. 7: shows the cross section B-B of the third bone transplant; and
- Fig. 8: shows a longitudinal section of a third bone transplant.

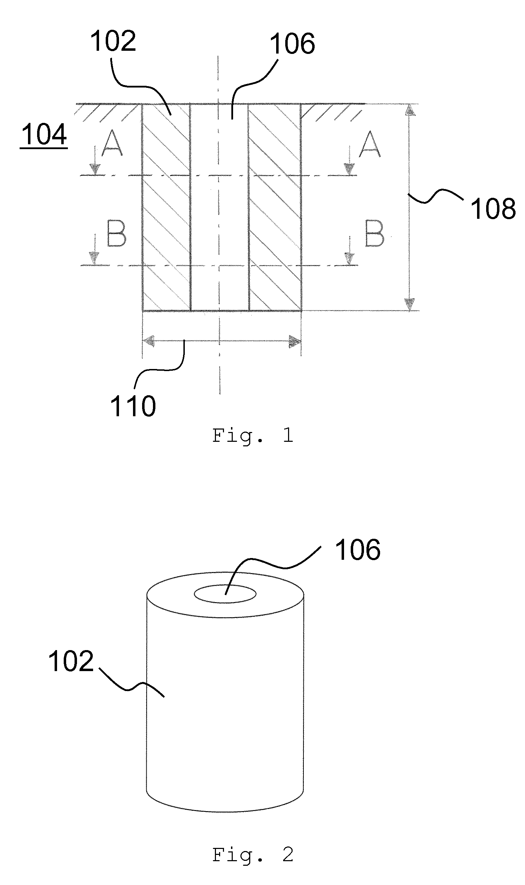

Fig. 1 shows a longitudinal section of a hollow-cylindrical bone transplant 102. The bone transplant 102 has been introduced into a jawbone 104. A clearance 106 extending in the middle of the bone transplant 102, along the longitudinal axis thereof, serves the purpose of receiving an implant.

Transplants 102 of the type represented in Fig. 1 may be part of a set with a plurality of bone transplants 102. In order to provide a respectively suitable bone transplant 102 for the augmentation of damaged locations of a jawbone 104, the heights 108 and/or the outside diameters 110 of the bone transplants 102 contained in the set vary. In particular, the set may contain bone transplants 102 with different outside diameters 110. This makes it possible for damaged locations of different extents to be augmented. For adaptation to the depth of the damaged location, the heights 108 of the bone transplants 102 contained in the set may also vary.

A first hollow-cylindrical bone transplant 102 with a longitudinal section that is represented in Fig. 1 is shown in Fig. 2.

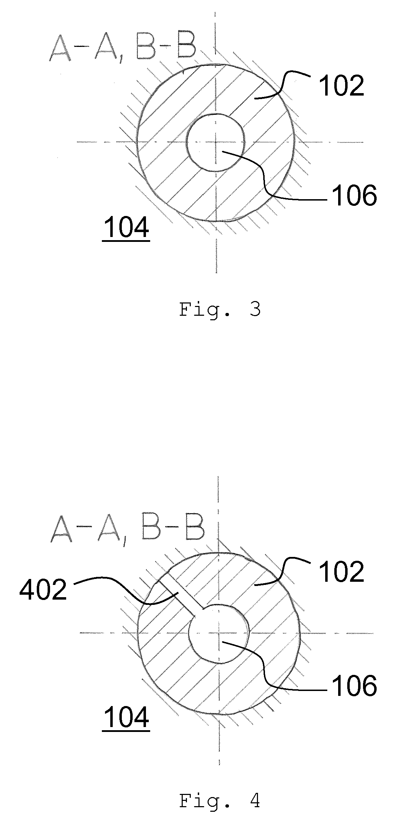

The cross sections A-A and B-B (cf. Fig. 1) of the first bone transplant 102 are represented in Fig. 3. The solid form of the wall of the bone transplant 102 can be seen here. As a result of this, the first bone transplant 102 cannot be made to expand when an implant is introduced. The outer form of the bone transplant 102 does not change when this happens. Therefore, a sufficiently load-bearing connection between the bone transplant 102 and the jawbone 104 only comes about when the bone transplant 102 has grown together with the jawbone 104.

The cross sections A-A and B-B (cf. Fig. 1) of a second bone transplant 102 are represented in Fig. 4. This is similar in longitudinal section to the first bone transplant 102. Therefore, the longitudinal sectional representation in Fig. 1 also applies to the second bone transplant 102.

As a difference from the first bone transplant 102, the second bone transplant 102 has an expansion joint 402. This extends along the longitudinal axis over the entire height 108 of the bone transplant 102. The length of the expansion joint 402 therefore corresponds to the height 108 of the bone transplant 102. The expansion joint 402 is correspondingly present not only in the cross sections A-A and B-B chosen by way of example but in all the cross sections of the second bone transplant 102.

The expansion joint 402 extends orthogonally in relation to the longitudinal axis, or within the plane of representation of Fig. 4, from the clearance 106 to the jawbone 104.

The expansion joint 402 enables the bone transplant 102 to expand when the surgeon introduces an implant into the clearance 106. When this happens, the outside diameter 110 of the bone transplant 102 increases. Similarly, the width of the expansion joint 402 increases. This is caused by the side surfaces of the bone transplant 102 that form the expansion joint 402 moving apart.

The expansion has the effect that the bone transplant 102 is pressed against the jawbone 104. As a result of this, a frictional connection between the bone transplant 102 and the jawbone 104 is formed. This improves the fixing of the bone transplant 102 in the jawbone 104 and consequently shortens the incorporation time, i.e. the time until the bone transplant 102 is certain to withstand loading.

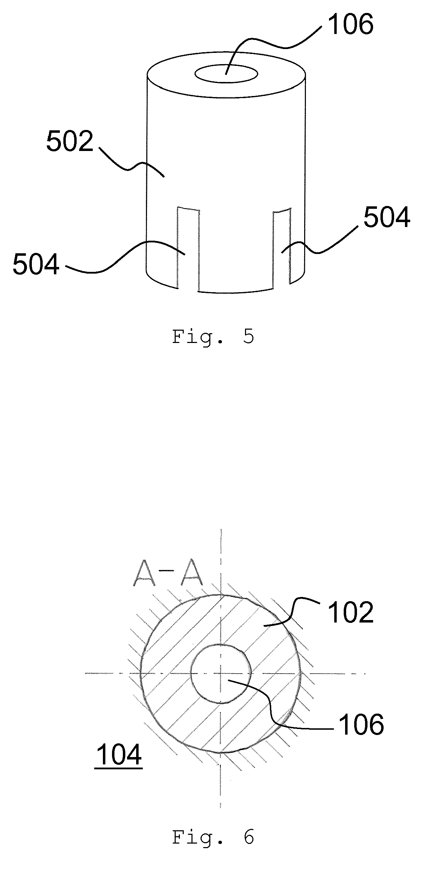

A third bone transplant 502, represented in Figs. 5 to 7, has four expansion joints 504. However, these do not extend over the entire height 108 of the bone transplant 102, as for instance in the case of the second bone transplant 102 (the bone transplant 102 would then be in four pieces). Instead, the length of the expansion joints 504 is smaller than the height of the bone transplant 502.

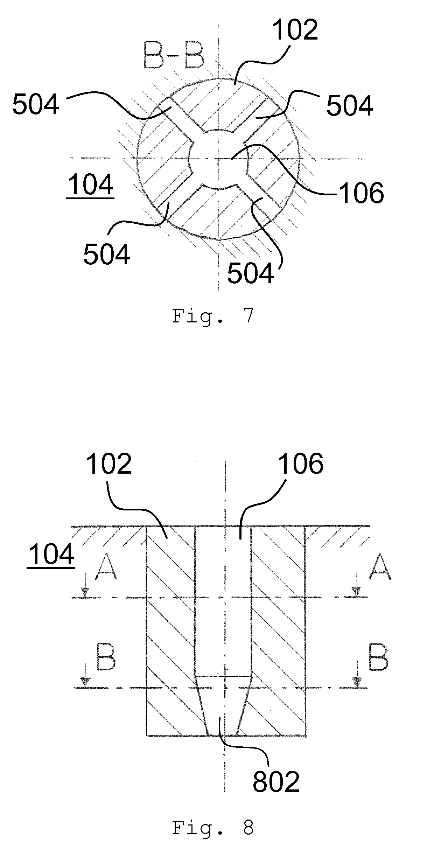

Figs. 6 and 7 illustrate this. In the cross section A-A, the profile of which is shown in Fig. 8, the bone transplant 502 is solid. Consequently, there is no expansion joint at the height of the cross section A-A.

By contrast, as can be seen in Fig. 7, the two expansion joints 504 extend through the cross section B-B (cf. Fig. 8). The expansion joints 504 end in a region located between the cross sections A-A and B-B (cf. Fig. 8). Consequently, the expansion joints 504 also extend through every cross section of the bone transplant 502 below this region. There is correspondingly no expansion joint in the cross sections of the bone transplant 502 above this region.

Due to the shortened expansion joints 504, the upper part of the bone transplant 502 cannot be expanded. The diameter of the implant is consequently restricted by the diameter of the clearance 106 in the upper part of the bone transplant 102. In order that the bone transplant 102 can nevertheless be made to expand in the lower part, the clearance 106 has a tapering region 802, as represented in Figure 8. In this region 802, the diameter of the clearance 106 is reduced from the top to the bottom (with respect to the representation of Figure 8). A screwed-in implant then makes the lower region of the bone transplant 502 expand like a straddling dowel.

### Reference numerals

- 102: Bone transplant
- 104: Jawbone
- 106: Clearance
- 108: Height
- 110: Outside diameter
- 502: Bone transplant
- 504: Expansion joint
- 802: Tapering region

### References Cited

### Non-patent literature

Aldredge W. A., Nejat R.: "Delayed implant procedure using de-proteinized bovine bone mineral: A report of 109 consecutive cases." in Compend. Contin. Educ. Dent. 2011 May; 32(4):66-71
Khoury F.: "Augmentation of the sinus floor with mandibular bone block and simultaneous implantation: a 6-year clinical investigation." in Int. J. Oral Maxillofac. Implants 1999 Jul-Aug; 14 (4) :557-64
Novell J., Novell-Costa F., Ivorra C., Fariñas O., Munilla A., Martinez C.: "Five-year results of implants inserted into freeze-dried block allografts." in Implant. Dent. 2012 Apr; 21(2):129-35
Gapski R., Neiva R., Oh T. J., Wang H. L.: "Histologic analyses of human mineralized bone grafting material in sinus elevation procedures: a case series." in Int. J. Periodontics Restorative Dent. 2006 Feb; 26(1):59-69

## Claims

1. Allogeneic or xenogeneic bone transplant (102; 502) with
a) a clearance (106), into which an implant can be introduced;
b) the bone transplant being formed such that it can be inserted into a jawbone (104) for augmentation; wherein
c) the bone transplant (102; 502) can bond to the surrounding jawbone (104) after being inserted; **characterised in that**
d) the bone transplant (102; 502) is designed such that it can be expanded by introducing the implant into the clearance (106).

2. Bone transplant (102; 502) according to the preceding claim,
**characterized by**
at least one expansion joint (402; 504),
the expansion joint (402; 504) being arranged such that the bone transplant (102; 502) can be expanded by introducing the implant into the clearance (106).

3. Bone transplant (102) according to the preceding claim,
**characterized**
**in that** the expansion joint (402) in one region completely separates the bone transplant (102) along the direction in which the implant is introduced.

4. Bone transplant (502) according to claim 2,
**characterized by**
four expansion joints (504),
wherein the four expansion joints (504) do not extend along the entire length of the bone transplant (502); and
wherein the expansion joints (504) begin from the end opposite to the opening for introducing the implant.

5. Bone transplant (502) according to one of the preceding claims,
**characterized**
**in that** the clearance (106) has a region (802) tapering in the direction in which the implant is introduced.

6. Bone transplant (502) according to the preceding claim,
**characterized**
**in that** at least one expansion joint (504) is formed, from the end opposite to the opening for introducing the implant, so as to begin at least partially in the tapering region (802).

7. Set with a plurality of allogeneic or xenogeneic bone transplants (102; 502) according to any of claims 1 to 6,
wherein the geometrical form of at least a first of the bone transplants (102; 502) differs from the geometrical form of at least a second of the bone transplants (102; 502).

8. Set according to the preceding claim,
**characterized in that**
the geometrical form of at least a first of the bone transplants (102; 502) represents a scaling of the geometrical form of at least a second of the bone transplants (102; 502).

## Patentansprüche

1. Allogenes oder xenogenes Knochentransplantat (102; 502) mit
a) einer Aussparung (106), in die ein Implantat eingebracht werden kann;
b) wobei das Knochentransplantat derart geformt ist, dass es zur Augmentation in einen Kieferknochen (104) eingesetzt werden kann;
c) wobei sich das Knochentransplantat (102; 502), nachdem es eingesetzt wurde, mit dem umgebenden Kieferknochen (104) verbinden kann;
**dadurch gekennzeichnet, dass**
d) das Knochentransplantat (102; 502) derart ausgestaltet ist, dass es durch Einbringen des Implantats in die Aussparung (106) aufgedehnt werden kann.

2. Knochentransplantat (102; 502) nach dem vorhergehenden Anspruch,
**gekennzeichnet durch**
mindestens eine Dehnungsfuge (402; 504),
wobei die Dehnungsfuge (402; 504) derart angeordnet ist, dass das Knochentransplantat (102; 502) **durch** Einbringen des Implantats in die Aussparung (106) aufgedehnt werden kann.

3. Knochentransplantat (102; 502) nach dem vorhergehenden Anspruch,
**dadurch gekennzeichnet,**
**dass** die Dehnungsfuge (402) das Knochentransplantat (102) entlang der Richtung, in welcher das Implantat eingeführt wird, in einem Bereich vollständig durchtrennt.

4. Knochentransplantat (502) nach Anspruch 2,
**gekennzeichnet durch**
vier Dehnungsfugen (504),
wobei die vier Dehnungsfugen (504) nicht entlang der vollständigen Länge des Knochentransplantats (502) verlaufen; und
wobei die Dehnungsfugen (504) an dem Ende, welches der Öffnung zum Einführen des Implantats gegenüber liegt, beginnen.

5. Knochentransplantat (502) nach einem der vorhergehenden Ansprüche,
**dadurch gekennzeichnet,**
**dass** die Aussparung (106) einen sich in Einführungsrichtung des Implantats verjüngenden Bereich (802) aufweist.

6. Knochentransplantat (502) nach dem vorhergehenden Anspruch,
**dadurch gekennzeichnet,**
**dass** mindestens eine Dehnungsfuge (504), von dem der Einführungsöffnung für das Implantat gegenüber liegenden Ende ausgehend, ausgebildet ist, so dass sie mindestens teilweise im sich verjüngenden Bereich (802) beginnt.

7. Satz mit einer Mehrzahl von allogenen oder xenogenen Knochentransplantaten (102; 502) nach einem der Ansprüche 1 bis 6,
wobei die geometrische Form mindestens eines ersten der Knochentransplantate (102; 502) sich von der geometrischen Form mindestens eines zweiten der Knochentransplantate (102; 502) unterscheidet.

8. Satz nach dem vorhergehenden Anspruch,
**dadurch gekennzeichnet,**
**dass** die geometrische Form mindestens eines ersten der Knochentransplantate (102; 502) eine Skalierung der geometrischen Form mindestens eines zweiten der Knochentransplantate (102; 502) darstellt.

## Revendications

1. Greffon osseux allogénique ou xénogénique (102 ; 502) comportant
a) un espace (106), dans lequel un implant peut être introduit ;
b) le greffon osseux étant formé de telle sorte qu'il peut être inséré dans un maxillaire (104) à des fins d'augmentation ;
dans lequel
c) le greffon osseux (102 ; 502) peut se lier au maxillaire qui l'entoure (104) après avoir été inséré ;
**caractérisé en ce que**
d) le greffon osseux (102 ; 502) est conçu de telle sorte qu'il peut être dilaté par introduction de l'implant dans l'espace (106).

2. Greffon osseux (102 ; 502) selon la revendication précédente,
**caractérisé par**
au moins un joint de dilatation (402 ; 504),
le joint de dilatation (402 ; 504) étant conçu de telle sorte que le greffon osseux (102 ; 502) peut être dilaté par introduction de l'implant dans l'espace (106).

3. Greffon osseux (102) selon la revendication précédente,
**caractérisé en ce que**
le joint de dilatation (402) dans une région sépare complètement le greffon osseux (102) le long de la direction dans laquelle l'implant est introduit.

4. Greffon osseux (502) selon la revendication 2,
**caractérisé par**
quatre joints de dilatation (504),
dans lequel les quatre joints de dilatation (504) ne s'étendent pas le long de la longueur totale du greffon osseux (502) ; et
dans lequel les joints de dilatation (504) débutent à l'extrémité opposée à l'ouverture destinée à l'introduction de l'implant.

5. Greffon osseux (502) selon l'une des revendications précédentes,
**caractérisé**
**en ce que** l'espace (106) comporte une région (802) s'effilant dans la direction dans laquelle l'implant est introduit.

6. Greffon osseux (502) selon la revendication précédente,
**caractérisé en ce que**
au moins un joint de dilatation (504) est formé, depuis l'extrémité opposée à l'ouverture destinée à l'introduction de l'implant, de sorte à débuter au moins partiellement dans la région d'effilement (802).

7. Ensemble comportant une pluralité de greffons osseux allogéniques ou xénogéniques (102 ; 502) selon l'une quelconque des revendications 1 à 6,
dans lequel la forme géométrique d'au moins un premier des greffons osseux (102 ; 502) diffère de la forme géométrique d'au moins un second des greffons osseux (102 ; 502).

8. Ensemble selon la revendication précédente,
**caractérisé en ce que**
la forme géométrique d'au moins un premier des greffons osseux (102 ; 502) représente une mise à l'échelle de la forme géométrique d'au moins un second des greffons osseux (102 ; 502).
